(19) Europäisches Patentamt
European Patent Office
Office européen des brevets

(11) Veröffentlichungsnummer: **0 282 902**
**A2**

# (12) EUROPÄISCHE PATENTANMELDUNG

(21) Anmeldenummer: **88103765.9**

(22) Anmeldetag: **10.03.88**

(51) Int. Cl.⁴: **C07C 47/228**, C07C 47/24, C07C 47/277, C07C 45/52, C07C 45/58

(30) Priorität: **18.03.87 DE 3708737**

(43) Veröffentlichungstag der Anmeldung: **21.09.88 Patentblatt 88/38**

(84) Benannte Vertragsstaaten: **BE CH DE FR GB IT LI NL**

(71) Anmelder: **BASF Aktiengesellschaft**
**Carl-Bosch-Strasse 38**
**D-6700 Ludwigshafen(DE)**

(72) Erfinder: **Hoelderich, Wolfgang, Dr.**
**Mannheimer Strasse 18 c**
**D-6710 Frankenthal(DE)**
Erfinder: **Goetz, Norbert, Dr.**
**Schoefferstrasse 25**
**D-6520 Worms 1(DE)**
Erfinder: **Recker, Hans-Gert, Dr.**
**Lisztstrasse 117**
**D-6700 Ludwigshafen(DE)**
Erfinder: **Smuda, Hubert, Dr.**
**Rahmengasse 32**
**D-6900 Heidelberg(DE)**

(54) **Verfahren zur Herstellung von Phenylacetaldehyden.**

(57) Gegenstand ist ein Verfahren zur Herstellung von Phenylacetaldehyden der Formel

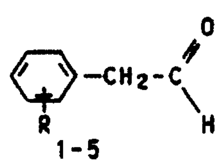

wobei R Alkyl-, Alkoxy-, Halogen-, Halogenalkyl-, Halogenalkoxy-und/oder Halogenalkylthio-Reste bedeuten bei dem man Epoxide der Formel

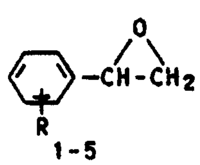

oder Phenylglykole der Formel

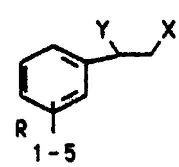

wobei R obige Bedeutung hat und Y bzw. X eine Hydroxygruppe bedeutet, an Siliciumdioxid-Katalysatoren, insbesondere mit Zeolithstruktur katalytisch umlagert.

EP 0 282 902 A2

## Verfahren zur Herstellung von Phenylacetaldehyden

Die Erfindung betrifft ein Verfahren zur Herstellung substituierter Phenylacetaldehyde, die als wertvolle Zwischenprodukte zur Herstellung neuer Wirkstoffe in Insektiziden dienen.

Es ist bekannt Phenylacetaldehyde auch in technischem Maßstab z.B. durch Dehydrierung von Phenylethanolen herzustellen. Bei diesem Verfahren erreicht man nur Teilumsatz bei verlustreicher Trennung von Ausgangs-und Endprodukt, da Phenylacetaaldehyde thermolabil sind. Bei der Fraktionierung kommt es zur Bildung von Selbstkondensationsprodukten. Auch die Herstellung von halogenhaltigen Phenylacetaldehyden ist auf diesem Wege nicht möglich, da unter den Reaktionsbedingungen Halogenabspaltung erfolgt. Auch durch die Umlagerung von Styroloxiden kann man zu den gewünschten Phenylacetaldehyden gelangen. In der Regel kommt es ebenfalls nur zu einem Teilumsatz und zur Bildung von - schwer abtrennbaren Nebenprodukten bei schlechten Selektivitäten und schlechten Standzeiten der bisher benutzten Katalysatoren aufgrund von Oberflächenbelegungen.

In EP 100 117 wird die Reaktion von Styroloxid und von am aromatischen Kern alkyl-bzw. alkoxylsubstituierten Styroloxiden an Titan-haltigen Zeolithen zu $\beta$-Phenylacetaldehyden in der flüssigen Phase bei 30 bis 100°C beschrieben. Der Katalysator muß aus schwer zugänglichen hochreinen Ausgangsstoffen wie Tetraalkylorthosilikat, Tetraalkylorthotitanat und Tetrapropylammoniumhydroxid hergestellt werden. Hoher Umsatz wird nur erzielt, wenn die Reaktion in einem Lösungsmittel wie Methanol oder Aceton bei 30 bis 100°C in der Flüssigphase stattfindet und Verweilzeiten von 1 bis 1,5 h eingehalten werden. Dies erschwert die Destillation. Weiterhin ist die Reaktion an Titan-haltigen Zeolithen nicht allgemein anwendbar und nur bei Styroloxid und am Aromaten alkylierten bzw. alkoxylierten Styroloxiden möglich.

Auch andere Verfahren zur Umlagerung von Epoxiden zu Carbonylverbindungen sind bekannt. Beispielsweise kann man Cyclododecanon an Pd-oder Rd-dotiertem $Al_2O_3$ aus Epoxycyclododecan erhalten. In dieser Arbeit wird ausdrücklich darauf hingewiesen, daß Zeolithe für diese Reaktion ungeeignet sind. Auch der Einsatz von A-Zeolithen für die Umlagerung von Butylenoxid zu Butyraldehyd (55 bis 72 %) ist beschrieben. Die Selektivität läßt noch zu wünschen übrig. Auch läßt sich der A-Zeolith-Katalysator nach seiner Desaktivierung durch Koks schwer regenerieren, da bei den hierfür notwendigen Temperaturen von etwa 500°C die Kristallstruktur dieses Zeolithen zerstört wird. Weiterhin ist es für die Umsetzung von Propylenoxid zu Aceton oder Propionaldehyd an alkali-dotierten X-Zeolithen notwendig, in Abwesenheit stark acider Zentren zu arbeiten.

Es ist auch bekannt, daß man Phenylacetaldehyde durch Umlagerung von Phenylglykol an Aluminiumsilikaten mit $SiO_2 : Al_2O_3 = 80 : 20 - 93 : 7$ gemischt mit z. B. Eisen-, Calcium-bzw. Magnesiumoxid oder an aktiviertem Ton in Suspension unter vermindertem Druck erhalten kann. Diesen beiden Verfahren ist gemeinsam, daß die Ausbeuten mit 50 bis 86 % noch verbesserungsbedürftig sind. Über die Standzeit und Regenerierbarkeit der Katalysatoren wird keine Aussage gemacht. Dieses Verfahren ist auch nicht flexibel und halogenierte Verbindungen erhält man nicht. Bei Ton handelt es sich um ein natürliches Mineral, das je nach Vorkommen unterschiedliche Zusammensetzung und damit unterschiedliche katalytische Eigenschaften und Selektivität aufweist. Dies erschwert insbesondere die Durchführung im kontinuierlichen technischen Verfahren.

Aldehyde kann man grundsätzlich auch in einer Rosenmund-Reduktion aus Carbonsäurechloriden gewinnen. Derartige Reaktionen verlaufen gut in der Flüssigphase mit Arylsäurechloriden. Bei anderen Säurechloriden wie z. B. Aralkylcarbonsäurechloriden erzielt man in der Regel niedrigere Ausbeuten durch Katalysatorvergiftung.

Die vorliegende Erfindung betrifft ein Verfahren zur Herstellung von Phenylacetaldehyden der Formel (I)

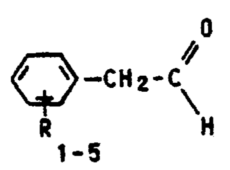

(I)

wobei R Alkyl-, Alkoxy-, Halogen-, Halogenalkyl-, Halogenalkoxy-und/oder Halogenalkylthio-Reste bedeuten und die erwähnten Nachteile vermieden werden, in dem man Epoxide der Formel (II) oder Phenylglykole der Formel (III)

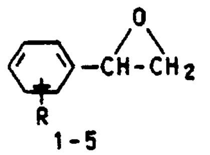 (II)

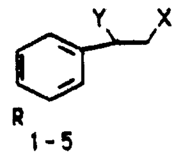 (III)

wobei R obige Bedeutung hat und Y bzw. X Hydroxygruppe bedeutet, an Siliciumdioxid-Katalysatoren katalytisch umlagert.

Vorteilhaft verwendet man bei dem erfindungsgemäßen Verfahren als Katalysator Siliciumdioxid mit Zeolithstruktur und als Ausgangsstoffe mit F und/oder Cl substituierte Epoxide oder Glykole und führt die Umlagerung in der Gasphase aus.

Das Verfahren bietet den Vorteil aus gut zugänglichen Ausgangsstoffen in Gegenwart von Katalysatoren, mit hoher Aktivität und leichter Regenerierbarkeit, bei langen Standzeiten bei hohen Umsätzen und hohen Selektivitäten die gewünschten Endprodukte herzustellen.

Weitere Vorteile der erfindungsgemäßen Umlagerung an diesen Katalysatoren sind: Vollständiger Umsatz, lange Standzeiten, hohe Selektivitäten und damit sehr gute Ausbeuten auch bei halogen-haltigen Ausgangsstoffen, einfache Isolierung der Endprodukte, in der Regel Weiterverwendung ohne zusätzliche Reinigung und leichte Regenerierbarkeit der Katalysatoren bei eventuell auftretender Verkokung.

Die erfindungsgemäße Umsetzung verläuft wie folgt:

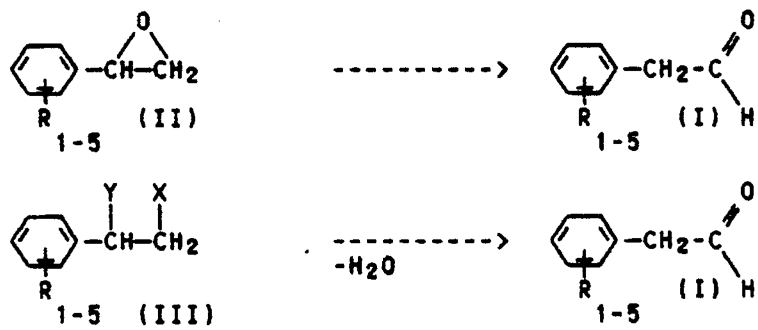

(II) (I)
(III) (I)

dabei kann R einen Alkyl-, Alkoxy-, Halogen-, Halogenalkyl-, Halogenalkoxy-und/ oder Halogenalkylthio-Rest bedeuten und Y und X für Hydroxy-Reste stehen.

Für das erfindungsgemäße Verfahren sind als Ausgangsstoffe z.B. folgende Epoxide geeignet: p-Fluorstyroloxid, p-Chlor-styroloxid, 2,4-Difluor-styroloxid, 3,4-Difluor-styroloxid, 2,4-Dichlor-styroloxid, 2,4,5-Trifluorstyroloxid, o-, m-, p-Trifluormethyl-styroloxid, o-, m, p-Methyl-styroloxid, 2,3,4,5-Tetrafluor-styroloxid, p-Trifluormethoxy-styroloxid, p-Trifluormethylthio-styroloxid, 2-Fluor-6-chlor-styroloxid, 2-Fluor-4-trifluormethylstyroloxid, 2-Fluor-4-trifluormethoxystyroloxid und 2-Methyl-4-fluor-styroloxid.

Die für das erfindungsgemäße Verfahren eingesetzten Phenylglykole sind z. B. Phenylglykol, p-Fluorphenylglykol, 2,4-Difluorphenylglykol, p-Chlorphenylglykol, p-Trifluormethylphenylglykol.

Als Katalysatoren für die erfindungsgemäße Umwandlung können gefällte Kieselsäuren aber auch durch Verbrennung von Siliciumhalogeniden hergestellte pyrogene Kieselsäuren eingesetzt werden. Diese Kieselsäuren lassen sich in bekannter Weise zu Formkörpern wie Strangpreßlinge, Tabletten, Ringe, Wabenkörper, Wagenrädern oder zu Wirbelgut verarbeiten.

Um eine möglichst hohe Selektivität, hohen Umsatz sowie lange Standzeiten zu erreichen, kann es vorteilhaft sein, die Siliciumdioxide zu modifizieren. Eine geeignete Modifizierung der Katalysatoren besteht darin, daß man die verformten oder unverformten Siliciumdioxide nach bekannten Methoden z. B. durch Tränken oder Aufsprühen mit Metallsalzen dotiert. Als Metalle sind Alkalimetalle wie Li, Cs, K, Erdalkalimetall wie Mg, Ca, Sr, Metalle der 3., 4. und 5. Hauptgruppe wie Al, Ga, Ge, Sn Pb, Bi, Übergangsmetalle der 4. - 8. Nebengruppe wie Ti, Zr, V, Nb, Cr, Mo, W, Mn, Re, Fe, Ru, Os, Co, Rh, Sr, Ni, Pd, Pt, Übergangsmetalle der 1. und 2. Nebengruppe wie Cu, Ag, Zn, Seltene Erdmetalle wie La, Ce, Pr, Nd, Er, Yb und U geeignet.

Eine mögliche Ausführungsform besteht z. B. darin, daß man $Cu(NO_3)_2$ $H_2O$ oder $Ni(NO_3)_2$ 6 $H_2O$ oder $Ce(NO_3)_3$ 6 $H_2O$ oder $La(NO_3)_2$ 6 $H_2O$ oder $Cs_2CO_3$ in Wasser löst und mit dieser Lösung das verformte oder unverformten Siliciumdioxid eine gewisse Zeit, z. B. 30 Minuten, tränkt. Die eventuell überstehende Lösung wird im Rotationsverdampfer von Wasser befreit. Danach wird das getränkte Material bei etwa

150°C getrocknet und bei etwa 550°C calciniert. Dieser Tränkvorgang kann mehrmals hintereinander vorgenommen werden, um den gewünschten Metallgehalt einzustellen.

Es ist auch möglich, eine wäßrige $Ni(CO_3)_2$-Lösung oder ammoniakalische $Pd(NO_3)_2$-Lösung herzustellen und darin das reine pulverförmige Siliciumdioxid bei 40 bis 100°C unter Rühren etwa 24 h aufzuschlämmen. Nach Abfiltrieren, Trocknen bei etwa 150°C und Calcinierung bei etwa 500°C kann das so gewonnene Material zu Strängen, Pellets oder Wirbelgut weiterverarbeitet werden.

Weitere Möglichkeiten der Modifizierung bestehen darin, daß man das Siliciumdioxid, verformt oder unverformt - einer Behandlung mit Salzsäure, Flußsäure, Schwefelsäure, Salpetersäure und/oder Wasserdampf unterwirft. Zweckmäßig wird das Siliciumdioxid mit Flußsäure, im allgemeinen mit 0,001 n bis 2 n, vorzugsweise 0,05 n bis 0,5 n Flußsäure behandelt, beispielsweise durch Erhitzen unter Rückfluß über einen Zeitraum von 0,5 bis 5, vorzugsweise 1 bis 3 Stunden. Nach Isolierung, z. B. durch Abfiltrieren und Auswaschen dieses Materials, wird dieses zweckmäßig, bei Temperaturen von 100 bis 160°C, getrocknet und bei Temperaturen von 450°C bis 600°C calciniert. Die Behandlung kann auch so vorgenommen werden, daß man auf das Siliciumdioxid nach seiner Verformung bei erhöhter Temperatur, zweckmäßig bei Temperaturen von 50 bis 90°C, vorzugsweise 60 bis 80°C, über einen Zeitraum von 0,5 bis 5 Std., vorzugsweise mit 12 bis 20 Gew.%iger Salzsäure, einwirken läßt. Zweckmäßig wird dieses Material anschließend ausgewaschen, bei Temperaturen von 100 bis 160°C getrocknet und bei Temperaturen von 450 bis 600°C calciniert. Einer HF-Behandlung kann sich auch eine HCl-Behandlung anschließen.

Weiterhin eignen sich als Katalysatoren für die erfindungsgemäße Umwandlung die Silicalite®, Molekularsiebe, sog. Silica Polymorphe. Silicalite lassen sich z. B. aus Kieselsolen in Gegenwart von Tetrapropylammoniumhydroxid und gegebenenfalls von Alkalihydroxid unter hydrothermalen Bedingungen bei Temperaturen von 150 bis 250°C herstellen. Die so hergestellten pulverförmigen Silicalite® können nach ihrer Isolierung, Trocknung bei 100 bis 160°C, vorzugsweise 110°C und Calcinierung bei 350 bis 550°C, vorzugsweise 450 - 500°C, mit einem Bindemittel im Verhältnis 90 : 10 bis 20 : 80 Gew.% zu Strängen oder Tabletten oder Wirbelgut verformt werden. Als Bindemittel eignen sich diverse Aluminiumoxide, bevorzugt Boehmit, amorphe Aluminosilikate mit einem $SiO_2/Al_2O_3$-Verhältnis von 25 : 75 bis 90 : 5, bevorzugt 75 : 25, Siliciumdioxid, bevorzugt hochdisperses $SiO_2$, Gemische auch hochdispersem $SiO_2$, Gemische aus hochdispersem $SiO_2$ und hochdispersem $Al_2O_3$, $TiO_2$, $ZrO_2$ sowie Ton. Nach der Verformung werden die Extrudate oder Preßlinge bei 110°C/16 h getrocknet und bei 500°C/16 h calciniert.

Man erhält auch vorteilhafte Katalysatoren, wenn der isolierte Silicalit® direkt nach der Trocknung verformt und erst nach der Verformung einer Calcinierung unterworfen wird. Die hergestellten pulverförmigen Silicalite® können auch in reiner Form, ohne Binder, als Stränge oder Tabletten oder Wirbelgut eingesetzt werden, wobei als Verstrangungs-oder Peptisierungshilfsmittel (Verformungshilfsmittel), z. B. Ethylcellulose, Stearinsäure, Kartoffelstärke, Ameisensäure, Oxalsäure, Essigsäure, Salpetersäure, Ammoniak, Amine, Silikoester und Graphit oder deren Gemische verwendet werden. Die Silicalite können wie oben beschrieben, z. B. mit Metallsalzen oder mit Säuren modifiziert werden, um eine möglichst hohe Selektivität, hohen Umsatz sowie lange Standzeiten zu erreichen. Die Silicalite können vorteilhafterweise mit Ammoniumionen behandelt, danach getrocknet und bei 450 - 550°C calciniert werden.

Wenn bei der erfindungsgemäßen Verwendung der Siliciumdioxid-Katalysatoren eine durch Koksabscheidung bedingte Desaktivierung eintritt, empfiehlt es sich, die Katalysatoren durch Abbrennen der Koksablagerung mit Luft oder mit einem Luft/$N_2$-Gemisch bei 400 bis 550°C, bevorzugt 500°C, zu regenerieren.

Durch partielle Verkokung (pre-coke) ist es möglich, die Acidität des Katalysators für ein Selektivitätsoptimum des gewünschten Reaktionsproduktes einzustellen.

Die hier beschriebenen Katalysatoren können wahlweise als 2 bis 4 mm Stränge oder als Tabletten mit 3 bis 5 mm Durchmesser oder als Pulver mit Teilchengrößen von 0,1 bis 0,5 mm oder als Wirbelkontakt oder als andere Formkörper eingesetzt werden.

Die für die erfindungsgemäße Umwandlung der Epoxide bzw. Glykole in der Regel gewählten Reaktionsbedingungen sind in der bevorzugten Gasphase 200 bis 500°C, vorzugsweise 200 bis 400°C, und eine Belastung WHSV = 0,1 bis 20 $h^{-1}$,bevorzugt 0,5 bis 5 h 1 (g Epoxide/g Katalysator und Stunde). Die Reaktion kann im Festbett oder Wirbelbett durchgeführt werden. Im allgemeinen steigt der Umsatz mit steigender Temperatur stark an, während die Selektivität in einem bestimmten Temperaturbereich nur wenig zurückgeht.

Auch ist es möglich, die Reaktion in der Flüssigphase (Suspension-, Riesel-oder Sumpffahrweise) durchzuführen.

Das Verfahren wird in der Regel bei Normaldruck oder je nach Flüssigkeit der Ausgangsverbindung bei vermindertem oder erhöhtem Druck durchgeführt, wobei die Durchführung vorzugsweise kontinuierlich erfolgt.

Schwerflüchtige oder feste Ausgangsstoffe werden in gelöster Form z. B. in THF-, Toluol-oder Petrolether-Lösung eingesetzt. Im allgemeinen ist auch eine Verdünnung mit Lösungsmitteln wie z. B. voranstehend genannt oder mit Inertgasen wie $N_2$, Ar, $H_2O$-Dampf möglich.

Nach der Umsetzung werden die entstandenen substituierten Phenylacetaldehyde in bekannter Weise, z. B. durch Destillation aus dem Reaktionsgemisch isoliert; nicht umgesetzte Ausgangsstoffe werden gegebenenfalls in die Umsetzung zurückgeführt. Eine direkte Weiterverarbeitung der Reaktionsprodukte ist aufgrund der sehr hohen Ausbeuten möglich. Bei dem erfindungsgemäßen Verfahren fallen bevorzugt die monomeren Verbindungen an. Sollten jedoch auch Oligomere z. B. trimere Phenylacetaldehyde gebildet werden, so können diese abgetrennt und zu den gewünschten Monomeren nach bekannten Methoden gespalten werden.

Die nach dem erfindungsgemäßen Verfahren zugänglichen Verbindungen sind wichtige Zwischenprodukte für biologisch aktive Verbindungen, z. B. Insektizide wie Resmethrin. Weiterhin kann man die Verbindungen in einfacher Weise zu Aminen, Alkoholen und Säuren nach üblichen Methoden, z. B. durch Oxidation mit Sauerstoff, durch katalytische Hydrierung oder aminierende Hydrierung weiterverarbeiten, wobei man wiederum wertvolle Zwischenprodukte für verschiedene wichtige Mehrstufen-Synthesen erhält.

Die Herstellung der Epoxide kann entweder durch Epoxidation der entsprechenden Styrole oder aus Halogenacetophenonen durch Hydrieren zu Chlor-oder Bromhydrinen und durch anschließenden Ringschluß im alkalischen Medium erfolgen. Durch Umsetzung der Epoxide mit Wasser, Alkoholen, Carbonsäuren oder Phenolen sind weitere Vorprodukte erhältlich, die sich zu Phenylacetaldehyden umlagern lassen.

Beispiele 1 bis 21

Die Reaktionen werden unter isothermen Bedingungen in einem Rohrreaktor (Wendel, 0,6 cm Innendurchmesser, 90 cm Länge) in der Gasphase mindestens 6 Stunden lang durchgeführt. Die Trennung und Charakterisierung der Reaktionsprodukte erfolgt nach üblichen Methoden. Die quantitative Bestimmung der Reaktionsprodukte und der Ausgangsstoffe erfolgte gaschromatographisch und durch CO-Zahl.

Die in den Beispielen verwendeten Katalysatoren für die Umwandlung von Epoxiden und Glykolen zu Phenylacetaldehyden sind:

Katalysator A D 11 - 11® $SiO_2$

Katalysator B

660 g Kieselsol (30 Gew.% $SiO_2$) werden mit 567 g wäßriger Tetrapropylammoniumhydroxid-Lösung (20 %ig) gemischt und in einem Autoklaven bei 200°C 72 h umgesetzt. Nach Abtrennen von der Mutterlauge wird bei 120°C getrocknet und bei 500°C/16 h calciniert. Das Diffraktogramm dieses Pulvers zeigt die für den Silicaliten® typischen Röntgenbeugungsmuster. Dieses Pulver wird zu 2 mm Strängen mit einem Verformungshilfsmittel verformt, bei 110°C getrocknet und bei 500°C/16 h calciniert. Diese Stränge werden mit einer 20%igen $NH_4Cl$-Lösung bei 80°C ionenausgetauscht, bis der Na-Gehalt 0,015 Gew.% (nach Trocknung bei 110°C und Calcination bei 500°C/5h) beträgt.

Katalysator C

168 g Tetrapropylammoniumhydroxid (20 %ig) werden mit 660 g Kieselsol (30 Gew.% $SiO_2$), 319 g $H_2O$ und 15,7 g NaOH gemischt. Diese Mischung wird unter Rühren 72 h bei 200°C in einem Autoklaven gehalten. Das entstandene Produkt wird von der Mutterlauge abfiltriert, gewaschen, bei 120°C getrocknet und bei 500°C/16 h calciniert. Das Diffraktogramm dieses Pulvers zeigt die für den Silicaliten® typischen Röntgenbeugungsmuster.

Dieser pulverförmige Silicalit® wird mit hochdispersem $SiO_2$ im Gewichtsverhältnis 70 : 30 zu 2 mm Strängen verformt. Diese Stränge werden bei 110°C getrocknet und bei 500°C/16 h calciniert. Danach erfolgt ein Ionenaustausch mit 20 %iger $NH_4Cl$-Lösung bei 80°C während 2 h. Der Na-Gehalt des Katalysators C beträgt dann 0,15 Gew.% nach Trocknung bei 110°C und Calcination bei 500°C/5 h.

Katalysator D (Vergleichskatalysator)

Boehmit wird zu 2 mm Strängen verformt, bei 110°C/16 h getrocknet und bei 500°C/16 h calciniert.

Katalysator E (Vergleichskatalysator)

$TiO_2$ P25® wird zu 2 mm Strängen verformt, bei 110°C/16 h getrocknet und bei 500°C/16 h calciniert.

Katalysator F (Vergleichskatalysator)

Nioboxydhydrat wird mit Boehmit im Gewichtsverhältnis 60 : 40 zu 2 mm Strängen verformt. Die Stränge werden bei 110°C getrocknet und bei 500°C/16 h calciniert.

Katalysator G (Vergleichskatalysator) ZnO R5-10®

Katalysator H (Vergleichskatalysator) $Al_2O_3$ D10-10®

Die mit diesen Katalysatoren erzielten Versuchsergebnisse sind in der Tabelle unter Beispiel 1 bis 19 zusammengestellt.

Beispiele 20 und 21

4-Fluorphenylglykol wird bei 300°C und WHSV = 3 $h^{-1}$ an den Katalysatoren A und B umgesetzt. Katalysator A liefert 84,2 % Selektivität und Katalysator B 85,8 % Selektivität jeweils bei 100 % Umsatz.

Tabelle

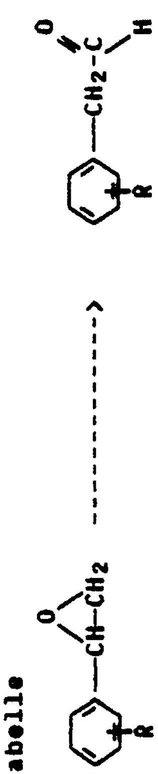

| Beispiel | 1 | 2 | 3 | 4 | 5 | 6 |
|---|---|---|---|---|---|---|
| Katalysator | A | A | A | A | A | A |
| R | 4-Fluor- | 2,4-Di-fluor | 3,4-Di-fluor | 2,4-Di 1) chlor | 3,4-Di 2) chlor | 4-Trifluor-2) methyl |
| Temperatur °C | 300 | 300 | 300 | 300 | 300 | 300 |
| WHSV $h^{-1}$ | 3 | 3 | 3 | 3 | 3 | 3 |
| Umsatz % | 100 | 100 | 100 | 100 | 100 | 100 |
| Selektivität % | | | | | | |
| Aldehyd | 87.3 | 82.9 | 80.7 | 83.0 | 85.6 | 89.5 |

1) gelöst in THF; 50 %ige Lösung
2) gelöst in Toluol; 50 %ige Lösung
3) Vergleichsbeispiele

Tabelle Forts.

| | | | | | | | |
|---|---|---|---|---|---|---|---|
| Beispiel | 7 | 8 | 9 | 10 | 11 | 12 | 13 |
| Katalysator | A | A | A | B | B | B | C |
| R | 2-Trifluormethyl | 2-Methyl-4-fluor | 4-Trifluormethoxy | 4-Fluor | 4-Trifluormethyl | 2-Methyl-4-fluor | 4-Fluor |
| Temperatur °C | 300 | 300 | 300 | 300 | 300 | 300 | 250 |
| WHSV $h^{-1}$ | 3 | 3 | 3 | 3 | 3 | 3 | 3 |
| Umsatz % | 100 | 100 | 100 | 100 | 100 | 100 | 100 |
| Selektivität % | | | | | | | |
| Aldehyd | 91,3 | 89,8 | 90,5 | 88,9 | 90,5 | 91,5 | 82,2 |

1) gelöst in THF; 50 %ige Lösung
2) gelöst in Toluol; 50 %ige Lösung
3) Vergleichsbeispiele

Tabelle Forts.

| Beispiel | 14 3) | 15 3) | 16 3) | 17 3) | 18 3) | 19 3) |
|---|---|---|---|---|---|---|
| Katalysator | D | D | E | F | G | H |
| R | 2-Trifluor-methyl | 4-Fluor- | 4-Fluor | 4-Fluor | 4-Fluor | 4-Fluor |
| Temperatur °C | 300 | 300 | 300 | 300 | 300 | 300 |
| WHSV $h^{-1}$ | 3 | 3 | 3 | 3 | 3 | 3 |
| Umsatz % | 100 | 100 | 95 | 100 | 79,5 | 100 |
| Selektivität % | | | | | | |
| Aldehyd | 65,9 | 60,5 | 59,2 | 68,3 | 72,5 | 62,1 |

1) gelöst in THF; 50 %ige Lösung
2) gelöst in Toluol; 50 %ige Lösung
3) Vergleichsbeispiel

## Ansprüche

1. Verfahren zur Herstellung von Phenylacetaldehyden der Formel (I)

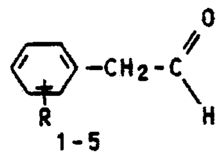

wobei R Alkyl-, Alkoxy-, Halogen-, Halogenalkyl-, Halogenalkoxy-und/oder Halogenalkylthio-Reste bedeuten; dadurch gekennzeichnet, daß man Epoxide der Formel (II) und/oder Phenylglykole der Formel (III)

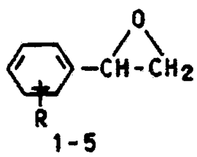 (II)

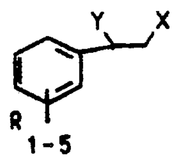 (III)

wobei R obige Bedeutung hat und Y bzw. X Hydroxygruppe bedeutet, an Siliciumdioxid-Katalysatoren katalytisch umlagert.

2. Verfahren nach Anspruch 1, dadurch gekennzeichnet, daß man als Katalysator Siliciumdioxid mit Zeolithstruktur verwendet.

3. Verfahren nach Anspruch 1, dadurch gekennzeichnet, daß man als Ausgangsstoffe bevorzugt mit F und/oder Cl substituierte Epoxide oder Glykole einsetzt.

4. Verfahren nach Anspruch, dadurch gekennzeichnet, daß man die Umlagerung in der Gasphase ausführt.